# EUROPEAN PATENT APPLICATION

(11) **EP 3 701 995 A1**
(43) Date of publication of application: **02.09.2020**
(21) Application number: 19159996.8
(22) Date of filing: 28.02.2019
(51) Int. Cl.: A61M 25/10, A61M 25/01, A61M 25/00

(54) **BALLOON CATHETER**

(71) Applicant: SIS Medical AG, 8500 Frauenfeld (CH)
(72) Inventor: Stöver, Michael, 8269 Fruthwilen (CH); Eckermann, Fabian, 8645 Jena (CH); Zwahlen, Willi, 67660 Betschdorf (FR)
(74) Representative: Keller & Partner Patentanwälte AG

(57) **Abstract**

The present application is drawn to a catheter (1), which has a proximal and a distal end. The catheter (1) comprises an elongated member (2) comprising an inner lumen (6) and a circumferential wall (5). The inner lumen is open at the distal end. At least one outer lumen (8) is arranged on an outside of said circumferential wall (5)and stretches from the proximal end to the distal end. The outer lumen (8) comprises an outer wall which is connected to the circumferential wall (5). The outer lumen (8) is closed at the distal end. An inflatable balloon (9) is arranged the outer lumen(8), said balloon (9) stretching from a distal to a proximal position (19,20). A reinforcement member (15) is arranged within said inner lumen (6) and stretches from the proximal end towards an end position (18) which is located proximal of the proximal position (20). An access opening (14) through said circumferential wall (5) is arranged at the end position. The access opening (14) allows the passage of a guide wire (16) into the inner lumen (6). The at least one outer lumen (8) is arranged only on a partial section of the circumference of the circumferential wall (5) at least in an area of said end position (18). The access opening (14) is further located on said circumferential wall (5) outside of said partial section.

## Description

### Technical Field

The invention relates to a catheter with a balloon which may be produced with a minimal amount of manufacturing steps and number of parts as well as a method for manufacturing such a balloon catheter.

### Background Art

Catheters as known in the art usually comprise an inflatable balloon arranged at a distal end of the catheter as well as a stiffer hypotube used to push the catheter through a body vessel of a patient. Further, such a catheter comprises a lumen for a fluid used to inflate the balloon as well as a lumen through which a guide wire may be inserted. The guide wire lumen remains open at a distal end of the catheter. The guide wire lumen may stretch through the entire catheter such that the guide wire may pass through the entire catheter (so-called over the wire catheter) or an exit port may be provided at a position proximal to the balloon such that the guide wire may exit the catheter via said exit port (so-called rapid exchange or monorail catheter).

Catheters as known in the art are typically assembled from six or more different parts in as many as forty manual assembly steps. This leads to high production costs, especially since each assembly step requires appropriate quality control.

The parts include tubes providing the lumen for the inflation fluid as well as the guide wire lumen, the hypotube, the balloon and the guide wire exit port as well as an adapter located at a proximal end of the catheter.

### Summary of the invention

It is the object of the invention to create a balloon catheter which is composed of a minimum number of parts and which may be produced by a minimal number of assembly steps.

The solution of the invention is specified by the features of claim 1. According to the invention the catheter, which has a proximal end and a distal end, comprises an elongated member stretching from the proximal end to the distal end, said elongated member comprises an inner lumen and a circumferential wall with a round cross-section. Said circumferential wall encloses the inner lumen which is open at the distal end. Further, at least one outer lumen is arranged on an outside of said circumferential wall and stretches from the proximal end to the distal end. The at least one outer lumen comprises an outer wall enclosing said at least one outer lumen. The outer wall is connected to the circumferential wall. An inflatable balloon is arranged on said elongated member and on said at least one outer lumen in a distal section of said catheter, said balloon stretching from a distal position to a proximal position. A volume of the inflatable balloon is in fluid connection with the outer lumen. An access opening through said circumferential wall is arranged at an end position which is located proximal of the proximal position. The access opening allows the passage of a guide wire into the inner lumen. The at least one outer lumen is arranged only on a partial section of the circumference of the circumferential wall at least in an area of said end position. The access opening is further located on said circumferential wall outside of said partial section.

By arranging the at least one outer lumen on the outside of the circumferential wall it is possible to form the circumferential wall as well as the outer wall of the at least one outer lumen at the same time by an extrusion process. Further, as the outer lumen does not extend around the entire circumference of the circumferential wall in the area of the end position, an access opening for a guide wire may be provided by simply cutting or piercing an opening into the circumferential wall in this area. This eliminates the necessity of providing a separate exit port and/or of appropriate welding seams.

The catheter according to the present invention is preferably used in percutaneous transluminal angioplasty (PTA) procedures, especially percutaneous transluminal coronary angioplasty procedures (PTCA). The balloon is thereby preferably used to widen a narrowed or obstructed artery or vein. Further, in alternative embodiments, the balloon may also be used to expand a stent mounted on the balloon.

The catheter preferably has a diameter of 0.2 - 1.2 mm, preferably of 0.5 - 0.8 mm. In the present application, the "catheter diameter" means the diameter of an imaginary circle enclosing both said circumferential wall of the inner lumen and said outer wall of said at least one outer lumen.

In the present application, the term "proximal end" means the end of the catheter which remains outside of a patient during a procedure. I.e. the proximal end is the end of the catheter pointing away of a treatment site, typically a narrowed or obstructed stretch of an artery or vein, during the procedure. Concurrently, the "distal end" is the end of the catheter which is introduced into a blood vessel of a patient to be treated. I.e. the distal end is the end of the catheter which will be positioned at the treatment site.

The inner lumen is open at the distal end such that a guide wire may be introduced into said inner lumen through this opening. Hence, the inner lumen serves the function of guidewire lumen.

Typically, in a PTA procedure, a guide wire is introduced first into a patient such that the guide wire spans through blood vessels from an incision area to the treatment site and unusually a short distance past said treatment site. A proximal end of the guide wire is then introduced into the inner lumen at the distal end of the catheter and the catheter is subsequently pushed over the guide wire until the distal end of the catheter reaches the treatment site.

The circumferential wall preferably has a wall thickness which is chosen such that the circumferential wall does not collapse during the procedure.

Preferably, the balloon is located a first distance from the distal end of the catheter on said distal section of the catheter. Hence, the catheter comprises a distal tip spanning from the distal end of the catheter to the balloon. Preferably, a hydrophilic coating is applied on the outer surface of the circumferential wall and the outer wall in the area of said distal tip. Preferably the first distance and hence the length of the distal tip is from 1 mm to 4 mm.

The balloon has a length which may vary depending on the intended use of the catheter. Preferably, the balloon has a length from 4 mm to 35 mm. The balloon spans from a distal position to a proximal position, wherein the length of the balloon equals the distance between said distal position and said proximal position.

The balloon preferably has a round cross-section with a diameter in the expanded state of preferably from 1 mm to 6 mm, more preferably from 1.5 mm to 4.5 mm. The balloon is preferably made of a polymer material and the wall thickness of the balloon material is chosen such that the balloon may withstand pressures of 10 bar to 40 bar. In the area of the distal position and of the proximal position the balloon preferably has conical sections where the diameter of the balloon is reduced, preferably to the diameter of the circumferential wall. The balloon may be formed by methods known in the art, e.g. by blow moulding.

The balloon encircles both the circumferential wall as well as the outer wall. This means that the circumferential wall and the outer wall are arranged within a volume of said balloon between the distal position and the proximal position. The balloon is connected to the circumferential wall and to the outer wall by a fluid-tight connection. Preferably the balloon is connected by means of welding or by means of an adhesive.

Preferably, the balloon is provided in a collapsed configuration and may be inflated by the introduction of a fluid, especially of a liquid, under pressure from the collapsed configuration into an inflated configuration. The at least one outer lumen is thereby preferably used as inflation lumen which conveys the fluid towards and from the balloon. Therefore, the outer wall preferably comprises at least one opening located between said proximal position and said distal position in order to allow a fluid exchange between the outer lumen and the volume of the balloon.

Preferably, the end position and the proximal position are spaced from each other by a second distance.

In the present application the "volume" of the balloon is understood as being the space enclosed by the inflatable balloon. The fluid connection allows a fluid provided through the outer lumen to enter into the volume of the inflatable balloon, e.g. for inflating the inflatable balloon. Likewise, in order to collapse the inflated balloon, a fluid present in the volume may be evacuated through the fluid connection and into the outer lumen. Hence, the lumen acts as so-called inflation lumen for the inflatable balloon.

The fluid connection between the outer lumen and the volume of the inflatable balloon is preferably realized by at least one opening provided on the outer wall between the proximal position and the distal position. In this case, the outer lumen is closed towards the distal end of the catheter, e.g. by means of a welding seam. Preferably, the outer lumen is closed by the same welding seam used to attach the inflatable balloon to the elongated member at the distal position.

In an alternatively preferred embodiment, the fluid connection is provided by removing the outer wall between the distal end and a termination position located between the proximal position and the distal positon, i.e. within the inflatable balloon. By removing the outer wall an open end of the outer lumen is provided within the inflatable balloon, said open end acting as fluid connection between the outer lumen and the volume of the inflatable balloon.

The access opening preferably stretches from the end position towards the distal end along a third distance. The access opening preferably is in the shape of a hole or slit. The dimensions of the access opening and especially the third distance are selected such that a guidewire may be introduced through said access opening and into or out of the inner lumen.

Specifically, the dimensions of the access opening are selected such as to allow a guidewire with a diameter of 0.1 mm to 0.4 mm to pass therethrough. In use, the catheter is pushed over a guidewire whose proximal tip is inserted into the inner lumen via the distal end. The proximal tip of the guidewire will reach the end position where it abuts on the reinforcement member. The reinforcement member preferably comprises a distal point which is formed such as to guide the proximal tip of the guide wire out through the access opening. With this configuration the catheter according to the present invention thus is a so-called rapid exchange or monorail catheter.

Preferably, the circumferential wall as well as the outer wall is made of a suitable polymer material, such as a polyamide, a polyamide block copolymer or polyethylene terephthalate.

Preferably, the at least one outer lumen is arranged on only said partial section of the circumference of the circumferential wall along the entire length of the catheter. With this arrangement, the production of the catheter is facilitated, since the elongated member comprising the circumferential wall and the outer wall of the at least one outer lumen may be extruded in a single step and used to assemble the catheter without any need of further modification of the elongated member.

Further, it is possible to produce catheters of varying lengths and with varying arrangements of the end position as the access opening may be cut or pierced at any location along the length of the elongated member.

Preferably, said outer lumen has a cross-section in the shape of a crescent. This configuration has the advantage that the outer lumen may be formed on the circumferential wall without increasing the overall diameter of the catheter too much. Further, the crescent shape allows withdrawing fluid from the balloon via the outer lumen by means of under pressure without the risk of a collapse of the outer lumen. Preferably, the outer wall has the shape of an ellipse cut in half along its smaller diameter.

Preferably, the elongated member comprises a reinforcement member, the reinforcement member stretching at least along a part of the distance between the proximal end and the end position. The reinforcement member increases the stiffness of the elongated tube at least along a section of the distance between the proximal end and the end position.

The reinforcement member preferably is in the form of a tube which is arranged over the circumferential wall and the outer wall. In an alternative preferred embodiment, the circumferential wall of the elongated element may be thicker in order to provide a reinforcement of the elongated element. In this case, the reinforcement member forms an integral part of the elongated member.

Between the end position and the distal end of the catheter no reinforcement member is present. Hence, this distal section of the catheter has a greater flexibility as the proximal section spanning from the proximal end to the end position.

Preferably, the reinforcement member is arranged within the inner lumen and stretches from the proximal end to the end position.

The reinforcement member preferably has a diameter which corresponds to an inner diameter of the circumferential wall. This way, the reinforcement member completely fills the inner lumen from the proximal end to said end position. The reinforcement member increases the stiffness of the inner lumen. Hence, the catheter according to the present invention does not need a hypotube.

The reinforcement member preferably comprises a distal point in the shape of a ramp, said reinforcement member being arranged within said inner lumen such that said ramp is pointing towards said access opening.

As understood herein, a "ramp" has an inclined surface or curved surface which is not symmetric relative to a length axis of the catheter. With other words, the distal point of the reinforcement member extends further in the direction of the distal end on a first side of the inner lumen as on a second side, which is located 180° from the first side. Said second side is preferably oriented towards the access opening, so that the inclined or curved surface of the ramp guides a guide wire impinging on said surface towards the access opening.

The distal point of the reinforcement member is preferably machined into the appropriate shape prior to the introduction of the reinforcement member into the inner lumen.

Preferably, the reinforcement member is made of at least one polymer material. When using a reinforcement member made of at least one polymer material it is possible to coextrude the reinforcement member at the same time as the elongated member is extruded, hence further facilitating the production of the catheter.

In a preferred embodiment, the reinforcement member has a stiffness which varies from the proximal end towards the end position. This allows adapting the stiffness of the proximal section of the catheter for different procedures or anatomical conditions.

Preferably, the stiffness of the reinforcement member decreases from the proximal end to the end position. This decrease is preferably constant. However, in certain embodiments, the decrease may be realized in a step-wise manner.

In order to vary the stiffness of the reinforcement member, two or more polymers each having a different stiffness may be mixed in varying ratios along the length of the reinforcement member, i.e. between the proximal end and the end position.

In an alternative embodiment, the reinforcement member is made of a metal or metal alloy. Hence, the reinforcement member may be a piece of wire which is cut into the correct length and introduced into the inner lumen, e.g. through the proximal end. Preferably, a distal end of said wire is ground or cut in the shape of the ramp.

Preferably, the catheter further comprises a radiopaque marker element. The radiopaque marker element may be arranged within said at least one outer lumen at a defined position. Hence, during a procedure, it is possible to locate and to determine the position of the catheter using medical imaging techniques, especially X-ray systems such as a C-arm.

Such as not to obstruct the outer lumen, e.g. when said outer lumen is being used as inflation lumen, the radiopaque element is preferably located in an area close to the distal end of the catheter.

In one embodiment, the radiopaque marker may be used to close the at least one outer lumen at the distal end of the catheter.

Preferably, the radiopaque marker element stretches at least partially from said distal position to said proximal position of the balloon. With this embodiment, it is possible for a surgeon to locate the position of the balloon during a procedure. The radiopaque member preferably does not stretch the entire distance between the distal position and the proximal position, such that an unobstructed transfer of a fluid from the outer lumen into the volume of the balloon and vice versa via an opening in the outer wall is possible.

In certain embodiments, said catheter comprises more than one outer lumen. Preferably, each outer lumen includes a separate outer wall. Alternatively, two or more outer lumen may include a single outer wall, each of the outer lumen being separated from neighbouring lumen by means of at least one longitudinal partitioning wall. Preferably, the catheter may comprise two, three or four outer lumen.

In the case that the catheter comprises more than one outer lumen, it is important to note that all outer lumen are configured such as to only occupy a partial section of the circumference of the circumferential wall in the area of the end position.

The present application further relates to a method of manufacturing a catheter. In a first step, an elongated member having a distal end and a proximal end is extruded. The elongated member has a circumferential wall with a round cross-section and an inner lumen enclosed by said circumferential wall, as well as at least one outer wall connected to an outside of said circumferential wall enclosing an outer lumen. The at least one outer wall is arranged only on a partial section of the circumference of said circumferential wall, said circumferential wall and said at least one outer wall being extruded together in a single step. In a next step, an inflatable balloon is arranged over said circumferential wall and said at least one outer wall in a distal section of the catheter, said balloon being positioned between the distal end and an end position located proximal of the distal end, the balloon stretching from a distal position to a proximal position being distal to the end position. Subsequently, a fluid communication between a volume of the inflatable balloon and of the outer lumen is created between the proximal position and the distal position. Then, the balloon is welded to the circumferential wall and to the outer wall at the distal position and at the proximal position. Finally, the circumferential wall is cut or pierced at the end position and outside of said partial section of the circumference in order to create an access opening through said circumferential wall allowing the passage of a guide wire into the inner lumen.

The method according to the present invention is preferably used to manufacture a catheter as described above.

The method therefore allows manufacturing a catheter with only a small number of manufacturing steps, which greatly reduces the cost and time required for manufacturing a catheter.

Preferably, an opening through the outer wall is produced between said distal position and said proximal position in order to create the fluid communication between the outer lumen and the volume of the inflatable balloon. This allows the passage of a fluid between the outer lumen and the volume of the inflatable balloon.

In an alternatively preferred embodiment, the outer wall is removed between the distal end and a termination position located between the proximal position and the distal positon, i.e. within the inflatable balloon. Removal of the outer wall may be e.g. be performed by cutting the outer wall away.

The reinforcement member is preferably introduced into the inner lumen via the proximal end.

Preferably, a reinforcement member is arranged within the inner lumen, the reinforcement member stretching from the proximal end to the end position.

Preferably, the reinforcement member is co-extruded into the inner lumen at the same time as the extrusion of the circumferential wall and said at least one outer wall. This further reduces the number of manufacturing steps, as the reinforcement member is produced at the same time as the circumferential wall and the outer wall.

Preferably, a flow rate of a polymer material for the co-extruded reinforcement member is gradually increased for a defined period of time once the extrusion of the circumferential wall and the at least one outer wall has reached the end position, such that a distal point of said reinforcement member is shaped in a ramp.

A radiopaque marker element is preferably introduced into said outer lumen from the first end prior to the welding step.

Preferably, an adapter is connected to the proximal end of the catheter, said adapter allowing coupling the catheter to further devices, preferably by means of a luer-type lock.

Other advantageous embodiments and combinations of features come out from the detailed description below and the entirety of the claims.

### Brief description of the drawings

The drawings used to explain the embodiments show:
- Fig. 1: a schematic drawing of an embodiment of an inventive catheter;
- Fig. 2: a cross-section of the elongated member;
- Fig. 3: a detailed view of the area at the end position;
- Fig. 4: the catheter of Fig. 1 along its entire length;
- Fig. 5: a schematic drawing of a further embodiment of a catheter according to the present invention along its entire length.

In the figures, the same components are given the same reference symbols.

### Preferred embodiments

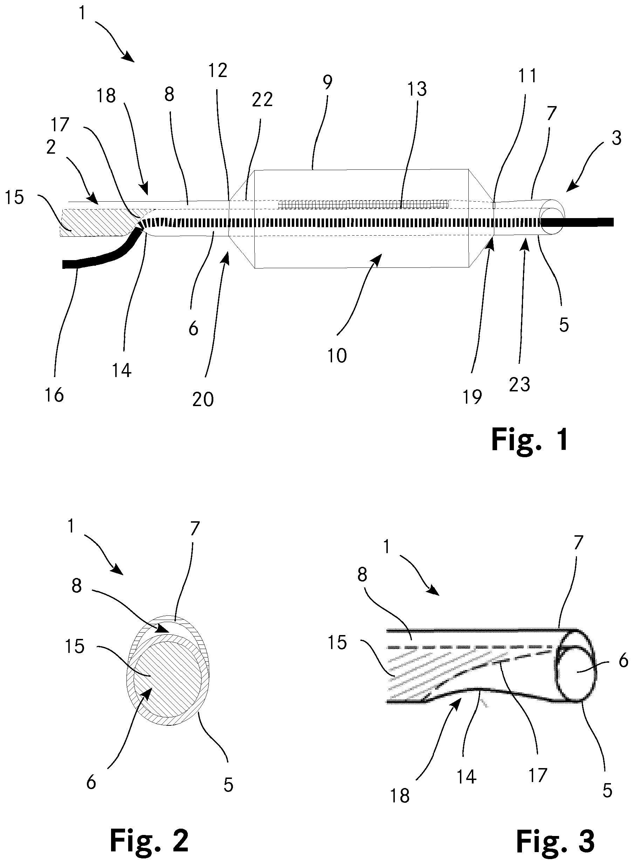

Fig. 1 shows a schematic drawing of an embodiment of a catheter 1 according to the present invention. The catheter 1 comprises an elongated member 2 which extends from a distal end 3 to a proximal end (not shown). The elongated member 2 comprises a circumferential wall 5 which encircles an inner lumen 6. The circumferential wall 5 has a round cross-section. Further, an outer wall 7 is arranged on an outside of the circumferential wall 5. The outer wall 7 defines an outer lumen 8.

A reinforcement member 15 is arranged in the first lumen 6. This reinforcement member 15 spans from the proximal end to an end position 18. The reinforcement member 15 may be made of a polymer material or of a wire of metal or metal alloy. The distance between the proximal end and the end position 18 constitutes a proximal section of the catheter 1. From the end position 18 to the distal end 3 the inner lumen 6 remains open. In the area of the end position 18 the circumferential wall 5 comprises an access opening 14. A guidewire 16 may be introduced through the distal end 3 into the inner lumen 6. Said guidewire 16 may exit the inner lumen 6 through the access opening 14. Hence, the catheter 1 may be used as so-called rapid exchange or monorail catheter. In the area of the access opening 14, the reinforcement member 15 has a distal point 17 which is shaped as a ramp pointing towards the access opening 14.

The distance between the end position 18 and the distal end 3 constitutes a distal section of the catheter 1. As there is no reinforcement member 15 present in the inner lumen 6 in the distal section, the catheter 1 is more flexible compared to the proximal section.

In this distal section the catheter 1 comprises an inflatable balloon 9 which is arranged on the circumferential wall 5 of said elongated member 2 and said outer wall 7. The inflatable balloon 9 spans from a distal position 19 to a proximal position 20. The distal position 19 is spaced from the distal end 3 by a first distance. Said first distance constitutes the distal tip 23 of the catheter 1. The proximal position 20 is spaced from the end position 18 by a second distance. Between the distal position 19 and the proximal position 20 a radiopaque marker element 13 is arranged within the outer lumen 8. The balloon 9 is welded to the circumferential wall 5 and to the outer wall 7 at a first welding seam 11 located at the distal position 19. Additionally, the outer lumen is sealed by this first welding seam 11 by welding the outer wall 7 to the circumferential wall 5. Hence, the outer lumen 8 is closed towards the distal end 3 of the catheter. The balloon 9 is further sealed by means of a second welding seam 12 to the outer wall 7 and to the circumferential wall 5 at the proximal position 20. In contrast to the distal position 19, the outer lumen 8 remains open at the distal position 19.

The inflatable balloon 9 comprises a volume which may be filed by a fluid in order to inflate said inflatable balloon 9. The fluid is provided from a fluid source (not shown) under pressure through the outer lumen 8. The outer lumen 8 hence serves as inflation lumen of the catheter 1. The outer wall 7 comprises an opening 22 through which the fluid may flow into the volume 10 of the balloon 9. The opening 22 is located distal of the proximal position 20 but proximal to the radiopaque marker element 13. By applying negative pressure on the outer lumen 8 the fluid may be withdrawn from the volume 10 hence leading to the collapse of the balloon 9.

Fig. 2 is a cross section of the elongated member 2. The cross section shown shows the elongate body 2 between the end position 18 and the proximal position 20. As may be seen, the circumferential wall 5 has a round cross-section which encircles the inner lumen 6 while the outer wall 7 is formed and arranged in such a way that the outer lumen 8 has a cross-section in the shape of a crescent. The reinforcement member 15 is arranged within the inner lumen 6 in the cross section shown.

Fig. 3 is a detailed view of the area of the end position 18. As may be seen, the distal point 17 of the reinforcement member 15 is shaped in the form of a ramp which is directed towards the access opening 14. This shape guides a guide wire which is pushed through the inner lumen 6 from the distal end 3 out of the access opening 14.

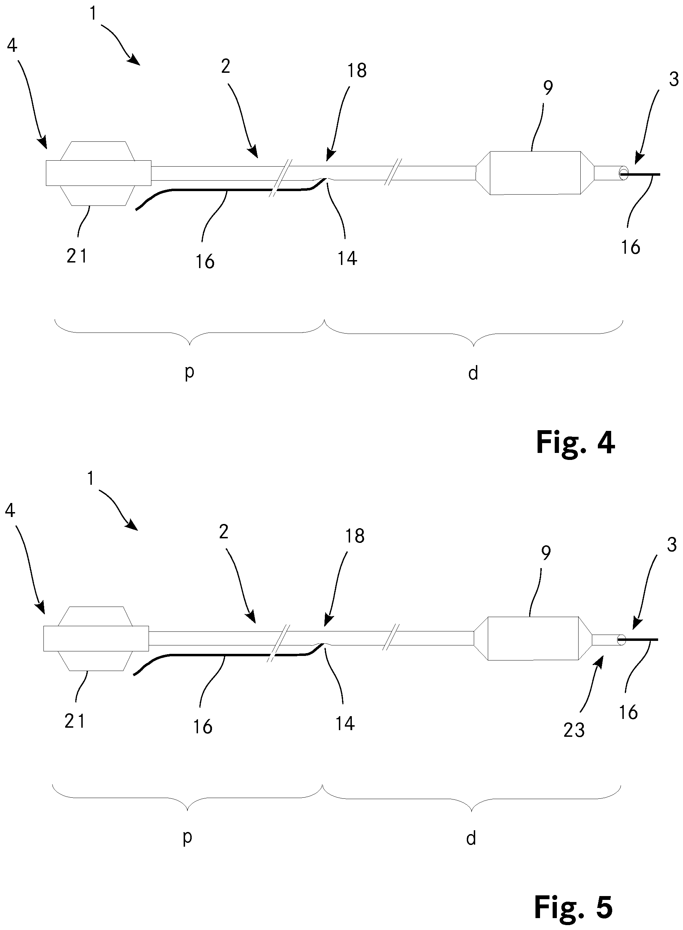

Fig. 4 shows the catheter 1 over its entire length. It is to be noted that the drawing is not to scale and that the catheter 1 would be considerably longer than shown. Hence, some parts are not shown, as is exemplified by the parallel bars.

The catheter 1 comprises an adapter 21 on a proximal end 4. The adapter 21 allows to connect the catheter 1 to a source of fluid (not shown), e.g. by means of a luer connection. In the figure the balloon 9 is shown in the expanded configuration. The guidewire 16 is introduced into the inner lumen 6 through the distal end 3 end exits through the access opening 14 located in the area of the end position 18. The proximal section p of the catheter 1 spans from the proximal end 4 to the end position 18 while the distal section d spans from the end position 18 to the distal end 3. As a reinforcement member 15 is arranged within the inner lumen 6 in the proximal section p this section is less flexible than the distal section d.

Fig. 5 shows an alternative embodiment of a catheter 1 according to the present invention over its entire length. As for Fig. 4, it is to be noted that the drawing is not to scale and that the catheter 1 would be considerably longer than shown. Hence, some parts are not shown, as is exemplified by the parallel bars.

In the embodiment as shown in Fig. 5 the outer wall 7 has been removed in the area of the distal tip 23 of the catheter 1 as well as over a part of the circumferential wall 5 of the elongated body 2 between the distal position 19 and the proximal position 20, i.e. within the volume of the inflatable balloon 9.

## Claims

1. Catheter with a proximal end and with a distal end, said catheter comprising:
a) an elongated member stretching from the proximal end to the distal end, said elongated member comprising an inner lumen and a circumferential wall with a round cross-section, said circumferential wall enclosing said inner lumen, the inner lumen being open at the distal end;
b) at least one outer lumen arranged on an outside of said circumferential wall and stretching from the proximal end to the distal end, said at least one outer lumen comprising an outer wall enclosing said at least one outer lumen and being connected to said circumferential wall;
c) an inflatable balloon arranged on said elongated member and on said at least one outer lumen in a distal section of said catheter, said balloon stretching from a distal position to a proximal position, a volume of the inflatable balloon being in fluid connection with the outer lumen;
**characterized in that**
an access opening through said circumferential wall is arranged at an end position which is located proximal of the proximal position, said access opening allowing the passage of a guide wire into the inner lumen, wherein the at least one outer lumen is arranged on only a partial section of the circumference of the circumferential wall at least in an area of said end position and said access opening is located on said circumferential wall outside of said partial section.

2. Catheter according to claim 1, **characterized in that** said at least one outer lumen is arranged on only said partial section of the circumference of the circumferential wall along the entire length of the catheter.

3. Catheter according to claim 2, **characterized in that** said outer lumen has a cross-section in the shape of a crescent.

4. Catheter according to any of claims 1 to 3, **characterized in that** the elongated member comprises a reinforcement member, said reinforcement member stretching at least along a part of the distance between the proximal end and the end position.

5. Catheter according to claim 4, **characterized in that** said reinforcement member is arranged within the inner lumen and stretches from the proximal end to the end position.

6. Catheter according to claim 5, **characterized in that** said reinforcement member comprises a distal point in the shape of a ramp, said reinforcement member being arranged within said inner lumen such that said ramp is pointing towards said access opening.

7. Catheter according to any of claims 4 to 6, **characterized in that** said reinforcement member is made of at least one polymer material.

8. Catheter according to claim 7, **characterized in that** said reinforcement member has a stiffness which varies from the proximal end towards the end position.

9. Catheter according to any of claims 4 to 6, **characterized in that** said reinforcement member is made of a metal or metal alloy.

10. Catheter according to any of claims 1 to 9, **characterized in that** the catheter further comprises a radiopaque marker element, said radiopaque marker element being arranged within said at least one outer lumen at a defined position.

11. Catheter according to claim 10, **characterized in that** said radiopaque marker element stretches at least partially from said distal position to said proximal position of the balloon.

12. Catheter according to any of claims 1 to 11, **characterized in that** said catheter comprises more than one outer lumen.

13. Method of manufacturing a catheter, preferably according to any of claims 1 to 13, comprising the following steps:
a) Extrusion of an elongated member having a distal end and a proximal end, said elongated member having a circumferential wall with a round cross-section and an inner lumen enclosed by said circumferential wall, as well as of at least one outer wall connected to an outside of said circumferential wall enclosing an outer lumen, said at least one outer wall being arranged only on a partial section of the circumference of said circumferential wall, said circumferential wall and said at least one outer wall being extruded together in a single step;
b) arranging a balloon over said circumferential wall and said at least one outer wall in a distal section of the catheter, said balloon being positioned between the distal end and an end point located proximal of the distal end, the balloon stretching from a distal position to a proximal position being distal to the end position and creating a fluid communication between a volume of the inflatable balloon and of the outer lumen between the proximal position and the distal position;
c) welding of said balloon to said circumferential wall and said outer wall at the distal position and at the proximal position;
d) cutting or piercing the circumferential wall at an end position being proximal to the proximal position and outside of said partial section of the circumference in order to create an access opening through said circumferential wall allowing the passage of a guide wire into the inner lumen.

14. Method according to claim 13, **characterized in that** a reinforcement member is arranged within the inner lumen, the reinforcement member stretching from the proximal end to the end position.

15. Method according to claim 14, **characterized in that** said reinforcement member is co-extruded into the inner lumen at the same time as the extrusion of the circumferential wall and said at least one outer wall.

16. Method according to claim 15, **characterized in that** a flow rate of a polymer material for the co-extruded reinforcement member is gradually increased for a defined period of time once the extrusion of the circumferential wall and the at least one outer wall has reached the end position, such that a distal point of said reinforcement member is shaped in a ramp.

17. Method according to any of claims 13 to 16, **characterized in that** a radiopaque marker element is introduced into said outer lumen from the first end prior to the welding step.

18. Method according to any of claims 13 to 17, **characterized in that** an adapter is connected to the proximal end of the catheter, said adapter allowing coupling the catheter to further devices, preferably by means of a luer-type lock.
